# EUROPEAN PATENT SPECIFICATION

(11) **EP 1 416 847 B1**
(45) Date of publication and mention of the grant of the patent: **14.03.2007**
(21) Application number: 02767596.6
(22) Date of filing: 31.07.2002
(51) Int. Cl.: A61B 5/0205, A61B 7/00

(54) **CARDIO-PULMONARY MONITORING DEVICE**
HERZ-LUNGEN-ÜBERWACHUNGSGERÄT
DISPOSITIF DE CONTROLE

(30) Priority: 31.07.2001 GB 0118728
(43) Date of publication of application: 12.05.2004
(73) Proprietor: Bluescope Medical Technologies Ltd, Belfast BT9 7BL (GB)
(72) Inventor: MURRAY, Jim, Belfast BT5 7BW (GB); DONNELLY, Peter, Castlewellan BT31 9RG (GB); FEE, John Patrick Howard, Belfast BT9 6JQ (GB)
(74) Representative: Casey, Lindsay Joseph
(86) International application number: PCT/GB2002/003526
(87) International publication number: WO 2003/011132

(56) References cited:
- WO-A-99/65379
- GB-A- 2 129 991
- GB-A- 2 240 392
- US-A- 5 844 997
- US-A- 6 102 856
- US-A- 6 139 505

## Description

The present invention relates to an apparatus for monitoring breath and heart sounds. In particular the apparatus allows continuous cardio-pulmonary monitoring.

Monitoring of breath and heart sounds is used both in diagnosis and as a means of determining the response of a patient to treatment.

Traditionally monitoring of breath and heart sounds has been effected by a stethoscope. However, there are many instances where the full use and capabilities of the traditional stethoscope are restricted. In particular there may be problems using the stethoscope when, access to the patient is restricted, as in intensive care or operating theatre situations. Also the nature of the patient's condition, for example extensive burns, traumatic injury or obesity can restrict access. Also, if the environment surrounding the patient is noisy (e.g. in ambulances, helicopters, military vehicles, ships, disaster sites etc.) it may be difficult to use a stethoscope effectively.

A further disadvantage of the traditional stethoscope is that it relies on the person using the stethoscope having sensitive hearing across the full frequency range. Interpretation of the sounds produced by a traditional stethoscope relies on the auditory performance of the user. As auditory performance often declines with age, older health professionals using a traditional stethoscope can find it more difficult to correctly interpret the heart and breath sounds produced by a patient. Furthermore, there may be important respiratory and cardiac sounds which are outside the normal auditory range and therefore undetectable by "traditional" stethoscopes.

Cardio-pulmonary monitoring of patients with time is important to determine the response of a patient to treatment and in some cases the progress of disease. Further, cardio-respiratory monitoring of patients at risk from acute illness, including infants and children at risk from Sudden Infant Death Syndrome (SIDS), may enable earlier treatment.

Monitoring of cardio-pulmonary function with time using a traditional stethoscope requires that the health professional is able to detect changes in the heart and breath sounds from individual measurements at particular time points such as each hour, day, week or longer. This relies on the ability of the health professional to recall what a previous measurement sounded like. In addition, if different health professionals are monitoring a patient's cardio-pulmonary function over a time period then the different interpretation of the sounds recorded by each health professional via a stethoscope means that subjective differences in the interpretation of a patient's cardio-respiratory sounds must be taken into account.

United States Patent Specification No. US-A-5 566 671 discloses a receptacle for a piezoelectric medical acoustic sensor comprising a lower side layer of a sound transmitting conformable material, preferably provided with an adhesive for attachment to the skin of a patient, and an outer sound deadening layer of an elastic foam material which prevents the transmission of room noise to the sensor. The outer layer is adhered to the skin side layer to form a pocket and the elastic properties of the outer layer hold the sensor in close acoustic contact with the skin side layer to improve the signal to noise ratio for the sensor.

International Patent Specification No. WO 99/65379 discloses an apron that independently performs ECG examinations, and other examinations. It can display a display screen installed on the apron. The apron can transfer data, and results, by means of cable or IR LED, to an outside instrument. The invention includes a twelve lead electrode apron that is put on a patient's body, a processing microcomputer unit that receives data from the electrodes, additional equipment installed on the examination apron, an optional display device, and cable or IR LED for transferring the information.

It is an object of the present invention to provide an improved apparatus for monitoring breath and heart sounds.

According to an aspect of the present invention, there is provided an apparatus as specified in claim 1.

An embodiment of the present invention will now be described, by way of example only, with reference to the accompanying drawings in which :-
Figure 1 shows a front view of the device according to the invention;
Figure 2 shows a side view of the device of figure 1 wherein the sensors are mounted in a matrix which is conjoined to an outer layer on one face and an adhesive layer on a second opposite face; and
Figure 3 shows a block diagram of the automatic respiratory recognition system.

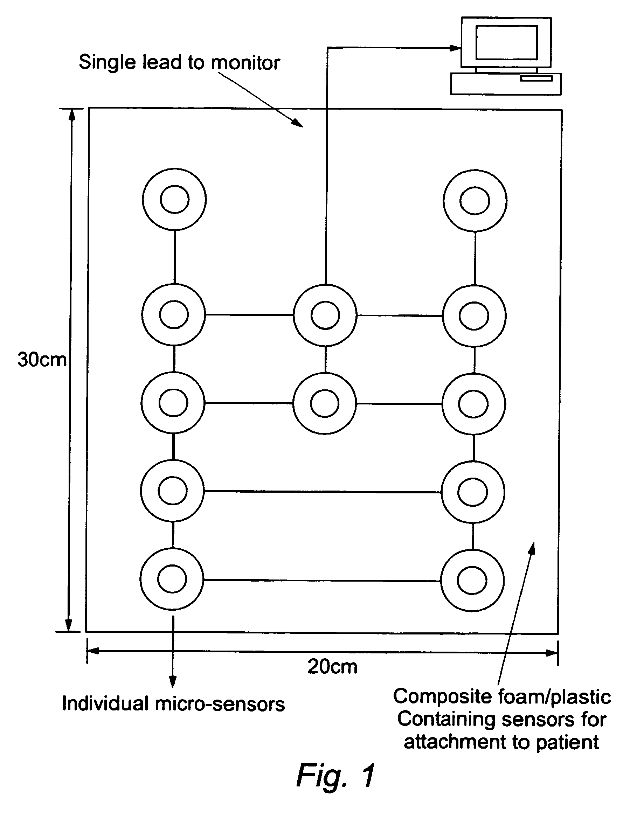

With reference to Figure 1 an embodiment of the present device is a pad comprising a plurality of sensors, typically between six to twelve sensors.

A plurality of sensors may be positioned within each region of the matrix pad with the intention being to capture the strongest "signal" from that region. The use of a plurality of sensors avoids the possibility of single sensor failure preventing measurement of the breath and heart sounds. Thereby accurate information may be relayed to the monitor.

The sensors are arrayed at particular locations in a matrix, the particular locations corresponding to appropriate anatomical positions to enable the continuous capture of breath and heart sounds.

The sensors will effectively "map" the lung and heart. Furthermore, the sensors in the device may capture important respiratory and cardiac sounds which are outside the normal auditory range and therefore undetectable by "traditional" stethoscopes.

The plurality of sensors will provide a complete lung/heart map. As an example if all is well with the patient the sensors will provide an all "green" display and if there are specific diseased areas "red" will be displayed within that region. There will also be varying shades of colour between these two ranges. It is also envisaged that a numerical display will be provided. For example, a range of 0-100, with 0 being the worst and 100 being the best. This may also be expressed as percent.

The pad comprising the matrix in which the sensors are arrayed is typically between 20 cm × 30 cm, however the size is dependent on the anatomical proportions of the patient. It can be envisaged that the size of the pad and the location of the sensors may be varied to suit babies or children.

The individual sensors are electronically connected such that the signals produced by each sensor can be transferred to a monitor by a single lead. The monitor enables the amplification, analysis and display of the signals produced by the sensors in both analogue and digital format.

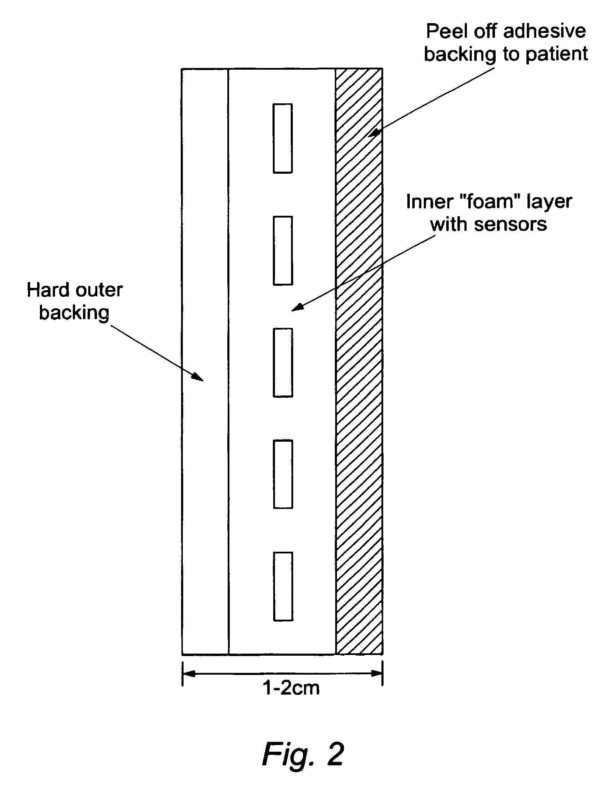

With reference to figure 2 the pad is comprised of multiple layers wherein a foam material layer houses the sensors. The foam material layer is attached to a first layer on one face and a second backing layer on the opposite face.

The first layer has an adhesive face, opposite the face of the first layer attached to the foam material layer, for fixing the pad to the patient and locating the sensors to suitable anatomical positions. The adhesive face of this first layer is protected by a peel off protective seal, which remains in place until the pad is to be positioned on to the patient. The adhesive used in the adhesive portion is preferably hypoallergenic, comfortable and sufficiently adherent to allow 2-5 days of continuous placement of the pad.

Between the first layer (in contact with the skin) and the second layer (containing the sensors) it is desirable to have an intermediate "space" or "vacuum" to facilitate and improve sound transmission from the chest to the sensors.

The second backing layer is attached to the foam material layer on the face opposite to that which is attached to the first layer. This second backing layer is thus the furthest from the patient when the pad is positioned on the patient in use. This second backing layer provides strength and robustness to the pad. Further, the second backing layer allows attachment of a lead to the pad for transfer of the signals produced by the sensors to a monitor.

Each sensor in the pad is electronically linked to a common lead for transfer of the signals produced by the sensors to a monitor.

Following the transfer of signals from each of the sensors by the common cable they are amplified, analysed and displayed in both analogue and digital format.

An alternative embodiment of the present invention is also provided wherein the device containing the sensors is not linked to a monitor by a cable, but by a wireless interface system. This wireless interface system allows remote or distant monitoring of the cardio-pulmonary signals.

Using the wireless interface, information can be relayed from the patient to a health professional without the need for the patient to be near a monitor or connected to any equipment other than the sensor containing device.

By suitable positioning of the pad incorporating the wireless interface onto the patient the cardio-pulmonary function of the patient may be monitored. This allows monitoring of patients' cardio-pulmonary function from their own homes, remote locations, or in situations where monitors are not be available, for instance in planes or at sea.

The apparatus can be used to effect the automatic recognition of respiratory sounds.

Respiratory sounds (normal and abnormal) have a typical frequency range of 100-2000Hz and a dynamic range of some 50-60dB. The upper extent of the frequency range is dependent upon the point at which the sound is transduced. The sound is effectively low-pass filtered by the body tissue between the lungs and the transducer, with the cut-off frequency of the low-pass fiiltering being dependent of the transducer site.

For digital processing, respiratroy signals should be sampled with a minimum sampling frequency of 4kHz at a minimum of 8 bits / sample. However, in system and algorithm development stages, a sampling frequeny of at least 8 khz at 16 bits / sample is recommended.

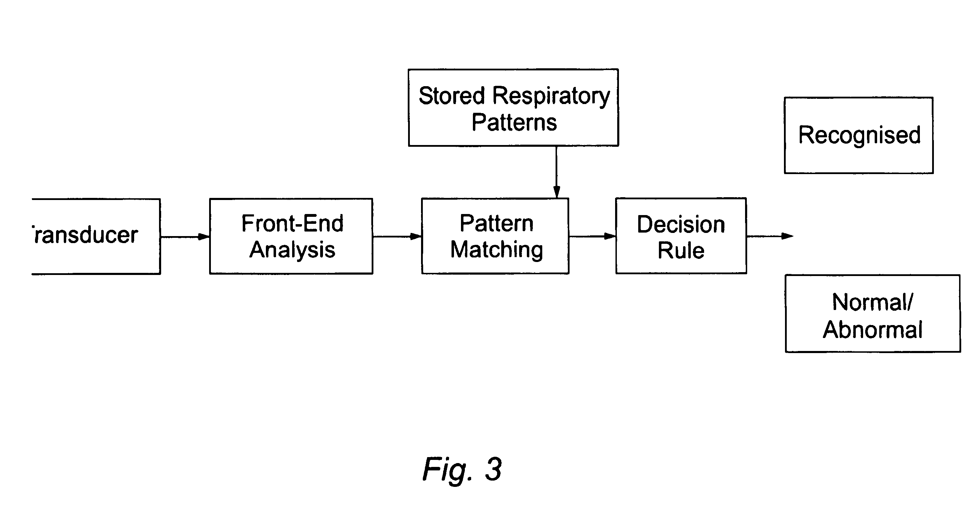

As shown in the block diagram of figure 3 of the automatic respiratory recognition system the objective is to automatically determine whether the input acoustic pattern is normal / abnormal and, if abnormal which pathological condition is determined.

The front end analysis involved in the canonic automatic respiratory recognition system is
(1) Bandpass filtering the signal in the range 10Hz-2kHz,
(2) Sub-band processing of the signal using two sub-bands - 10 to fn and fn to 2kHz, where fn is the anticipated upper frequency limit of the normal range of sound from the transducer site,
(3) Identification and rate of respiratory inhalation and exhalation phases,
(4) Short-term spectral / parametric analysis of respiratory phases in both sub-bands,

The pattern classifier comprises pattern matching against stored respiratory patterns (based on possible spectral, energy or parametric information) and a decision rule, which may be linear or nonlinear. The pattern classifier can be either a standard statistical classifier or a classifier based on artificial intelligence techniques, such as neural networks or fuzzy logic classifiers.

In use the recorded sounds are transmitted to the analysis means are band pass filtered and sub-band pass filtered.

The recorded sounds also include sounds which are detected and then analysed in real time.

The filtered data is then compared against previously determined data using the pattern classifier.

The previously determined data can be from the same or different patient and may comprise a description indicating if the predetermined data is indicative of normal of abnormal breath and heart sounds.

The newly recorded data can thus be compared against the predetermined data and assigned as normal or abnormal. Further comparison of the recorded data signal with abnormal data might allow a match against a similar previously determined pattern, and such a match may allow a diagnosis of the abnormality and possibly the disease promoting the abnormality to be made by the analysis means.

A global positioning satellite locator (GPS) or further sensors enabling monitoring of the patient may also be incorporated into the pad of the device and the information from the GPS locator or alternative sensor relayed to the monitor by the wireless interface means.

It can be envisaged that the device may be suitably positioned to the patient by alternative means than adhesive.

The sensors may be incorporated into a pad which can be wrapped around the patient or worn by the patient to allow positioning of the sensors at suitable anatomical positions.

Alternatively the sensors may be incorporated with alternative fixing means such as suction cups to allow their accurate placement onto the patient.

The present invention has a number of advantages. It may be used to continuously monitor a patient's cardio-pulmonary function. This is advantageous over traditional stethoscopes, which can only record a patient's cardio-pulmonary function at distinct time points.

As the device allows the non-subjective monitoring of a patient's cardio-pulmonary function over time, differences in the interpretation of cardio-pulmonary sounds by different health professionals do not have to be taken into account when monitoring the patient.

The device is primarily designed for monitoring breath and heart sounds over the patient's chest, however it could easily be adapted for foetal monitoring either throughout pregnancy or during labour. Similarly, if a woman requires anaethesia/surgery / intensive care during her pregnancy it is not inconceivable that one device could be used to monitor the mother and another to monitor the foetus.

In use the device, which includes in the sensors is a pad which can be wrapped around the patient, the pad is then suitably positioned around the patient chest such that breath and heart sounds can be measured. Due to the plurality of the sensors the exact positioning of the pad is not crucial as typically if placed in a generally correct position, breath and heart sounds will be detected and recorded.

The pad is kept in position for a period of time suitable to allow data collection, this may be minutes, hours or days as required to allow breath and heart sounds to be suitably recorded.

The breath and heart sounds are transmitted to recording means to record the sounds.
Transmission may occur via wires linking the device to the recording and analysis means of via a wireless system.

Further usage and development of the device could be in the field of veterinary obstetrics and veterinary medicine with regard to both large and small animals that are pregnant/about foal, calf etc. or need anaesthesia and surgery.

The device will further provide clear and effective training for students of medicine and nursing, as it will allow the unambiguous interpretation of normal and pathological heart and breath sounds.

The device is robust, easily stored and not easily damaged. The entire device or any part thereof may also be disposable.

It maybe used in daylight or in the dark which is useful in military situations or for use in dark rooms.

As there is an equal distribution of sensors between the left and right sides of the chest, differential interpretation of normal and abnormal breath sounds will be possible.

The device will allow diagnosis or determination of a patient's response to treatment to be performed by a suitable health professional from a distance.

This distant or remote monitoring of a patient's cardio-pulmonary function has particular importance in cases where patients are in planes, ambulances, helicopters or remote situations.

## Claims

1. An apparatus comprising a device including a sound sensor embedded in a pad capable of attachment to the skin of a patient, the sensor being suitably positioned in the pad to allow the capture of breath and heart sounds over a period of time, means for recording the heart and breath sounds, and means to analyze the heart and breath sounds, **characterised in that** the apparatus comprises at least two sound sensors embedded at different locations in the same pad.

2. An apparatus as claimed in claim 1 wherein the device comprises at least two sensors positioned such that they are capable of being located one on each side of the patient's chest.

3. An apparatus as claimed in claim 1 wherein a plurality of sensors is suitability positioned for capturing breath and heart sounds by locating the sensors in a matrix.

4. An apparatus as claimed in claim 1, 2 or 3 wherein the pad is attachable to the patient's skin by adhesive means.

5. An apparatus as claimed in any preceding claim, wherein the pad may be worn or wrapped around a patient to suitability locate the sensors.

6. An apparatus as claimed in any preceding claim wherein the sensors are electronically connected to each other.

7. An apparatus as claimed in any preceding claim wherein the signals produced by the plurality of sensors are transferred to a monitor by a single cable.

8. An apparatus as claimed in any preceding claim wherein the sensors can be used remotely from the recording means and means to analyze the breath and heart sounds.

9. An apparatus as claimed in any preceding claim wherein the means for recording the breath and heart sounds can convert the breath and heart sounds into an analogue signal.

10. An apparatus as claimed in claims 1 to 8 wherein the means for recording the breath and heart sounds can convert the breath and heart sounds into a digital signal.

11. An apparatus as claimed in any preceding claim wherein the means to analyze the breath and heart sounds includes means for bandpass filtering the signal in the range 10Hz to an upper frequency limit.

12. An apparatus as claimed in any preceding claim wherein the means to analyze the breath and heart sounds includes means for sub-band processing the signal.

13. An apparatus as claimed in any preceding claim wherein the means to analyze the breath and heart sounds is capable of identifying the rate of respiratory inhalation and exhalation phases.

14. An apparatus as claimed in any preceding claim wherein the means to analyze the breath and heart sounds includes a pattern classifier to enable the signals recorded to be matched to previously determined breath and heart signals.

15. An apparatus as claimed in any preceding claim wherein the means to analyze the breath and heart sounds uses short term spectral/parametric analysis.

16. An apparatus as claimed in any preceding claim including a global positioning satellite locator.

17. An apparatus as claimed in any preceding claim including additional sensors for monitoring the physiological state of the patient.

18. A method for interpreting breath and heart sounds using the apparatus of claim 1, comprising the steps of:
i) positioning the device including the sensors around the area of interest,
ii) recording the breath and heart sounds over time,
iii) converting the breath and heart sounds to a signal in the range of 10Hz to an upper frequency limit,
iv) bandpass filtering the signal
v) identifying the rate of respiratoryinhalation and exhalation phases
vi) comparing the recorded signal data with known signal data of breath and heart sounds,
vii) determining if the signal data of breath and heart sounds recorded matches known signal data of breath and heart sounds,

19. A method as claimed in claim 18 including the step of sub-processing the recorded signal.

20. A method as claimed in claim 18 or 19 including the step of mapping the signals to the heart and lung.

## Patentansprüche

1. Gerät mit einer Vorrichtung, die einen Geräuschsensor eingebettet in einer Kompresse, die an der Haut eines Patienten befestigt werden kann, wobei der Sensor geeignet in der Kompresse positioniert ist, um das Erfassen von Atem- und Herzgeräuschen über eine Zeitspanne zuzulassen, ein Mittel zum Aufzeichnen der Herz- und Atemgeräusche und ein Mittel zum Analysieren der Herz- und Atemgeräusche einschließt, **dadurch gekennzeichnet, dass** das Gerät mindestens zwei Geräuschsensoren eingebettet an verschiedenen Stellen in der selben Kompresse aufweist.

2. Gerät nach Anspruch 1, bei dem die Vorrichtung mindestens zwei so positionierte Sensoren aufweist, dass sie jeweils auf einer Seite des Brustkorbs eines Patienten angeordnet werden können.

3. Gerät nach Anspruch 1, bei dem eine Mehrzahl von Sensoren geeignet zum Erfassen von Atem- und Herzgeräuschen durch Anordnen der Sensoren in einer Matrix positioniert ist.

4. Gerät nach Anspruch 1, 2 oder 3, bei dem die Kompresse an der Haut eines Patienten durch Klebemittel befestigt werden kann.

5. Gerät nach einem vorhergehenden Anspruch, bei dem die Kompresse um einen Patienten herum getragen oder gewickelt werden kann, um die Sensoren geeignet anzuordnen.

6. Gerät nach einem vorhergehenden Anspruch, bei dem die Sensoren elektrisch miteinander verbunden sind.

7. Gerät nach einem vorhergehenden Anspruch, bei dem die durch die Mehrzahl von Sensoren erzeugten Signale zu einem Monitor durch ein einzelnes Kabel übertragen werden.

8. Gerät nach einem vorhergehenden Anspruch, bei dem die Sensoren entfernt von dem Aufzeichnungsmittel und Mittel zum Analysieren der Herz- und Atemgeräusche verwendet werden können.

9. Gerät nach einem vorhergehenden Anspruch, bei dem das Mittel zum Aufzeichnen der Atem- und Herzgeräusche die Atem- und Herzgeräusche in ein Analogsignal umwandeln kann.

10. Gerät nach den Ansprüchen 1 bis 8, bei dem das Mittel zum Aufzeichnen der Atem- und Herzgeräusche die Atem- und Herzgeräusche in ein Digitalsignal umwandeln kann.

11. Gerät nach einem vorhergehenden Anspruch, bei dem das Mittel zum Analysieren der Atem- und Herzgeräusche Mittel zum Bandpassfiltern des Signals im Bereich von 10 Hz bis zu einer oberen Frequenzgrenze einschließt.

12. Gerät nach einem vorhergehenden Anspruch, bei dem das Mittel zum Analysieren der Atem- und Herzgeräusche Mittel für Unterbandverarbeitung des Signals aufweist.

13. Gerät nach einem vorhergehenden Anspruch, bei dem das Mittel zum Analysieren der Atem- und Herzgeräusche die Rate von Einatmungs- und Ausatmungsphasen identifizieren kann.

14. Gerät nach einem vorhergehenden Anspruch, bei dem das Mittel zum Analysieren der Atem- und Herzgeräusche einen Musterzuordner einschließt, um Übereinstimmen der aufgezeichneten Signale mit früher bestimmten Atem- und Herzsignalen zu ermöglichen.

15. Gerät nach einem vorhergehenden Anspruch, bei dem das Mittel zum Analysieren der Atem- und Herzgeräusche Kurzzeitspektral-/Parameteranalyse verwendet.

16. Gerät nach einem vorhergehenden Anspruch, das ein GPS-Suchgerät einschließt.

17. Gerät nach einem vorhergehenden Anspruch, das zusätzliche Sensoren zum Überwachen des physiologischen Zustands des Patienten einschließt.

18. Verfahren zum Interpretieren von Atem- und Herzgeräuschen unter Verwendung des Geräts von Anspruch 1, das die folgenden Schritte aufweist:
i) Positionieren der die Sensoren einschließenden Vorrichtung um den Bereich von Interesse herum,
ii) Aufzeichnen der Atem- und Herzgeräusche im Verlauf der Zeit,
iii) Umwandeln der Atem- und Herzgeräusche in ein Signal im Bereich von 10 Hz bis zu einer oberen Frequenzgrenze,
iv) Bandpassfiltern des Signals,
v) Identifizieren der Rate von Einatmungs- und Ausatmungsphasen,
vi) Vergleichen der aufgezeichneten Signaldaten mit bekannten Signaldaten von Atem- oder Herzgeräuschen,
vii) Bestimmen, ob die Signaldaten von aufgezeichneten Atem- und Herzgeräuschen mit bekannten Signaldaten von Atem- und Herzgeräuschen übereinstimmen.

19. Verfahren nach Anspruch 18, das den Schritt einschließt, Unterverarbeitung des aufgezeichneten Signals auszuführen.

20. Verfahren nach Anspruch 18 oder 19, das den Schritt einschließt, die Signale zum Herz und zur Lunge abzubilden.

## Revendications

1. Un appareil comportant un dispositif comprenant un capteur de sons incorporé à un coussinet pouvant s'attacher à la peau d'un patient, le capteur étant placé dans le coussinet de manière à permettre la prise des sons respiratoires et cardiaques pendant une période de temps, un moyen d'enregistrer les sons respiratoires et cardiaques, et un moyen d'analyser les sons respiratoires et cardiaques, **caractérisé en ce que** l'appareil comporte au moins deux capteurs de son incorporés en des emplacements différents dans le même coussinet.

2. Un appareil conforme à la revendication 1, où le dispositif comporte au moins deux capteurs placés de manière à être capables d'être situés un de chaque côté du thorax du patient.

3. Un appareil conforme à la revendication 1, où une pluralité de capteurs est placée de manière à prendre les sons respiratoires et cardiaques en situant les capteurs dans une matrice.

4. Un appareil conforme à la revendication 1, 2 ou 3, où le coussinet peut s'attacher à la peau du patient par un moyen adhésif.

5. Un appareil conforme à une quelconque des revendications précédentes, où le coussinet peut être porté ou enroulé autour d'un patient pour placer les capteurs de manière appropriée.

6. Un appareil conforme à une quelconque des revendications précédentes, où les capteurs sont connectés les uns aux autres par voie électronique.

7. Un appareil conforme à une quelconque des revendications précédentes, où les signaux produits par la pluralité de capteurs sont transférés à un moniteur par un seul câble.

8. Un appareil conforme à une quelconque des revendications précédentes, où les capteurs peuvent s'utiliser à distance du moyen d'enregistrement et des moyens d'analyse des sons respiratoires et cardiaques.

9. Un appareil conforme à une quelconque des revendications précédentes, où le moyen d'enregistrer les sons respiratoires et cardiaques peut convertir les sons respiratoires et cardiaques en un signal analogique.

10. Un appareil conforme aux revendications 1 à 8, où le moyen d'enregistrer les sons respiratoires et cardiaques peut convertir les sons respiratoires et cardiaques en un signal numérique.

11. Un appareil conforme à une quelconque des revendications précédentes, où le moyen d'analyser les sons respiratoires et cardiaques comporte un moyen de filtrage passe-bande du signal dans la gamme 10 Hz jusqu'à une limite de fréquence supérieure.

12. Un appareil conforme à une quelconque des revendications précédentes, où le moyen d'analyser les sons respiratoires et cardiaques comporte un moyen de traitement en sous-bandes du signal.

13. Un appareil conforme à une quelconque des revendications précédentes, où le moyen d'analyser les sons respiratoires et cardiaques est capable d'identifier la fréquence des phases respiratoires d'inhalation et d'exhalation.

14. Un appareil conforme à une quelconque des revendications précédentes, où le moyen d'analyser les sons respiratoires et cardiaques comporte un classeur de formes pour permettre aux signaux enregistrés d'être appariés à des signaux respiratoires et cardiaques précédemment déterminés.

15. Un appareil conforme à une quelconque des revendications précédentes, où le moyen d'analyser les sons respiratoires et cardiaques utilise une analyse à court terme spectrale/paramétrique.

16. Un appareil conforme à une quelconque des revendications précédentes, comportant un système de positionnement mondial par satellite.

17. Un appareil conforme à une quelconque des revendications précédentes, comportant des capteurs supplémentaires pour surveiller l'état physiologique du patient.

18. Un procédé pour interpréter les sons respiratoires et cardiaques à l'aide de l'appareil de la revendication 1, comportant les étapes suivantes :
i) positionnement de l'appareil comportant les capteurs autour de la zone d'intérêt,
ii) enregistrement des sons respiratoires et cardiaques pendant un certain temps,
iii) conversion des sons respiratoires et cardiaques en un signal dans la gamme allant de 10 Hz jusqu'à une limite de fréquence supérieure,
iv) filtrage passe-bande du signal,
v) identification de la fréquence des phases respiratoires d'inhalation et d'exhalation,
vi) comparaison des données des signaux enregistrés avec des données connues de signaux de sons respiratoires et cardiaques,
vii) détermination de si les données de signaux des sons respiratoires et cardiaques enregistrés s'apparient à des données connues de signaux de sons respiratoires et cardiaques.

19. Un procédé conforme à la revendication 18, comportant l'étape de sous-traitement du signal enregistré.

20. Un procédé conforme à la revendication 18 ou la revendication 19, comportant l'étape de mise en correspondance des signaux avec le coeur et les poumons.
